# EUROPÄISCHE PATENTSCHRIFT

(11) **EP 0 583 717 B1**
(45) Veröffentlichungstag und Bekanntmachung des Hinweises auf die Patenterteilung: **18.03.1998**
(21) Anmeldenummer: 93112770.8
(22) Anmeldetag: 10.08.1993
(51) Int. Cl.: G01N 33/78, G01N 33/96

(54) **Verfahren und Standardlösung zur Bestimmung von Thyroxin (T4) oder Trijodthyronin (T3)**
Process and standard solution for the determination of thyroxine (T4) or triiodothyronine (T3)
Procédé et solution standard pour la détermination de thyroxine (T4) ou triiodothyronine (T3)

(30) Priorität: 14.08.1992 DE 4226949
(43) Veröffentlichungstag der Anmeldung: 23.02.1994
(73) Patentinhaber: BOEHRINGER MANNHEIM GMBH, 68298 Mannheim (DE)
(72) Erfinder: Lenz, Helmut Dr., D-82327 Tutzing (DE); Bialk, Peter Dr., D-82327 Tutzing (DE); Hoyle, Nicholas Dr., D-82327 Tutzing (DE)

(56) Entgegenhaltungen:
- EP-A- 0 337 467
- CHEMICAL ABSTRACTS, Band 89, Nr. 13, 25. September 1978, Columbus, Ohio, USA B. J. VAN DER WALT et al. "Bovine thyroxine-binding globulin. Purification and comparison of molecular weight and amino acid composition with human thyroxine- binding globulin." Seite 294, Zusammenfassung- -Nr. 102 271u

## Beschreibung

Die Erfindung betrifft ein Verfahren zur Bestimmung von Thyroxin (T₄) oder Trijodthyronin (T₃) im Serum unter Verwendung einer Standardlösung sowie diese Standardlösung.

Die Thyreoidhormone liegen im Blut weitgehend in proteingebundener Form vor. Das wichtigste Trägerprotein ist dabei das Thyroxin-bindende Globulin, das als TBG bezeichnet wird, an das ca. 80 % des gesamten im Blut vorliegenden Thyroxins und Trijodthyronins gebunden sind. Etwa 5 bis 10 % des gesamten Thyroxins liegen an Albumin gebunden vor und etwa 10 bis 15 % des gesamten Thyroxins liegen an Präalbumin gebunden vor. Die Gesamtheit dieser Proteine wird auch als Thyroxin-bindendes Protein (TBP) bezeichnet. Nur sehr kleine Anteile an Thyroxin und Trijodthyronin liegen im Blut in freier Form vor, in der Regel sind es ca. 0,03 % des T₄ bzw. 0,3 % des T₃. Nur die freigesetzten Hormone sind physiologisch wirksam, während die gebundenen Hormone T₃ und T₄ als metabolisch inerte Transportform im Blut zirkulieren und als Puffer zur Regelung des Spiegels dienen. Es ist daher wichtig, sowohl den Gesamtthyroxingehalt als auch den Anteil an freiem Thyroxin sowie freiem Trijodthyronin im Blut zu bestimmen, da dieser den funktionellen Thyreoidstatus unabhängig von Änderungen in der Konzentration oder Sättigung des Thyroxin-bindenden Proteins darstellt.

Es sind nun verschiedene Verfahren zur Bestimmung von Gesamt-thyroxin (T₄), freiem Thyroxin (FT₄) und Trijodthyronin (T₃) bekannt. Eine Schwierigkeit, die bei allen diesen bekannten Verfahren besteht, ist die Bereitstellung eines zur Eichung geeigneten Standards.

Für die Eichung ist es notwendig, Humanserumstandards mit bestimmten Thyroxin bzw. Trijodthyroninkonzentrationen und einem definierten FT₄- bzw. FT₃-Gehalt herzustellen. Dazu wurde bereits Humanserum als Ausgangsmaterial verwendet. Dem Humanserum mußte zuerst alles Thyroxin bzw. Trijodthyronin, sowohl in freier als auch in gebundener Form, entzogen werden und dann das so behandelte Humanserum mit definierten Thyroxin- oder Trijodthyroninmengen wieder aufgestockt werden. In der Literatur sind hierzu verschiedene Methoden beschrieben, beispielsweise die Behandlung mit Aktivkohle, mit Ionenaustauschern oder mit trägergebundenen Anti-T₄- bzw. -T₃-Antikörpern. Diese Verfahren sind alle sehr aufwendig. Außerdem besteht ein Problem darin, daß jedes Humanserum eine andere Zusammensetzung aufweist und daher die einzelnen Chargen große Schwankungen zeigen, so daß die Reproduzierbarkeit zu wünschen übrig läßt.

Ein weiterer Nachteil dieser bekannten Standardlösungen für die Bestimmung von T₃ und T₄ liegen darin, daß diese Lösungen, die die Zusammensetzung von Humanserum haben, nicht längere Zeit gelagert werden können, da einerseits das enthaltene Protein nach und nach denaturiert wird und andererseits sich das eingestellte Gleichgewicht zwischen gebundenem und freiem T₃ und T₄ verschiebt.

Aus der EP-A 0 337 467 ist es bekannt, zur Lösung der erwähnten Probleme eine Standard lösung für ein Verfahren zur Bestimmung von Thyroxin oder Trijodthyronin zu verwenden, die noch bei längerer Lagerung stabil ist und in einfacher Weise unter Verwendung relativ billiger Ausgangsmaterialien hergestellt werden kann, indem man zur Eichung mindestens eine Standardlösung verwendet, die TBG und Thyroxin bzw. Trijodthyronin gelöst in einer Pufferlösung enthält.

Nunmehr wurde gefunden, daß eine weitere sehr erhebliche Verbesserung der Lagerstabilität erreicht werden kann, wenn man in den vorstehend erwähnten Verfahren TBG aus RinderSerum verwendet.

Die Erfindung betrifft daher ein Verfahren zur Bestimmung von Thyroxin (T₄) oder Trijodthyronin (T₃) im Serum, wobei man zur Eichung eine Standardlösung verwendet, die Thyroxin-bindendes Globulin (TBG) und Thyroxin bzw. Trijodthyronin gelöst in einer Pufferlösung enthält, daß dadurch gekennzeichnet ist, daß man Rinder-TBG verwendet.

Während bei den bekannten Verfahren die Standardlösung als flüssiges Reagenz die Mindestlagerfähigkeit von 8 Wochen nur knapp erreicht und daher in lyophilisierter Form gelagert werden muß, ist die erfindungsgemäß verwendete Standardlösung in flüssiger Form mehr als 10-fach so lange lagerstabil. Nach den bis jetzt vorliegenden Ergebnissen erreicht die Lagerstabilität in flüssiger Form mindestens 18 Monate und zeigt nach Ablauf dieser Zeit immer noch volle Bindungsfähigkeit für T₄ und T₃, während die entsprechende Standard lösung mit Human-TBG selbst bei Lagerung unterhalb 10°C schon nach 12 Wochen um 20 % falsch zu hohe Werte liefert. Zudem ist das zur Gewinnung von Rinder-TBG verwendete Rinderserum wesentlich besser erhältlich als Humanserum.

Die überlegene Stabilität der erfindungsgemäßen Standardlösung ist aus der Tabelle 1 zu entnehmen. Man erkennt daraus, daß bei Human-TBG (H-TBG) die Analytstabilität rasch abnimmt, so daß falsch zu hohe Werte gefunden werden, die schon nach 3 Monaten 20 % höher sind (d.h. 120 % des Ausgangswertes) als bei einer frischen Lösung. Bei der erfindungsgemäßen Standard lösung hingegen ist auch nach 12 Monaten noch keinerlei Veränderung feststellbar, d.h. die Analysenwerte sind unverändert richtig bei 100 %. Die angegebene Prozentgehalte betreffen die gemessene Wiederfindung an Analyten im Vergleich zu einem frischen Produkt. Zu beachten ist hierbei, daß an TBG gebundener Analyt, also T₄ oder T₃, ja überhaupt nicht gefunden wird und ein Anstieg des Wertes für T₄ bzw. T₃ bedeutet, daß sich die Bindung an TBG verschlechtert hat, also falsche Werte im Vergleich zum frischen natürlichen TBG liefert. Die gerade noch akzeptable Grenze ist eine Veränderung um ± 5 %.

Zur Bestimmung von Thyroxin und Trijodthyronin sind verschiedene Verfahren bekannt. Bestimmungsverfahren werden beispielsweise beschrieben in Nuc. Compact 16 (1985), 321-327, J. Clin. Immunoassay 7 (1984), 192-205, und J. Clin. Chem. Clin. Biochem. 22 (1984), 895-904. Insbesondere werden zur Bestimmung der Thyreoidhormone Bestimmungsverfahren nach dem Prinzip des Immunoassays verwendet.

Bei einem bekannten Verfahren zur Bestimmung von Thyroxin oder Trijodthyronin im Serum nach dem Prinzip des Immunoassays konkurrieren in der Regel markiertes T₄ bzw. T₃ und T₄ bzw. T₃ der Probe um einen Bindepartner, wie beispielsweise einen Anti-T₄-Antikörper. Durch Einstellen der Reaktionsbedingungen, wie Konzentration einzelner Komponenten, Inkubationsdauer usw., kann entweder das Gesamtthyroxin, beziehungsweise Gesamttrijodthyronin oder das freie Thyroxin bzw. Trijodthyronin bestimmt werden. Dazu wird die Menge von an den Anti-T₄- bzw. -T₃-Antikörper gebundenem markiertem T₃ oder T₄ bzw. nicht gebundenem markiertem T₃ oder T₄ über die an sich bekannten Nachweisreaktionen bestimmt. Der Gehalt an T₃ oder T₄ kann dann nach der Messung der Markierung errechnet werden, indem man den erhaltenen Wert in Beziehung setzt zu Werten, die man mit bekannten T₃- oder T₄-Konzentrationen erhalten hat. Dazu ist es notwendig, eine Eichkurve zu erstellen, in der das Meßsignal (z.B. der Extinktionswert) gegen die T₃ oder T₄-Konzentration aufgetragen ist. Im Falle des Thyroxins erstellt man zweckmäßigerweise eine Eichkurve aus sechs Thyroxinlösungen mit verschiedenen Konzentrationen, da es keine lineare Beziehung gibt, die eine Zweipunkt-Standardmessung zulassen würde.

Das erfindungsgemäße Verfahren ist sowohl für die Bestimmung von freiem Thyroxin (FT₄), freiem Trijodthyronin (FT₃) als auch von Gesamt-Thyroxin (T₄) und Trijodthyronin (T₃) im Serum geeignet.

Das Thyroxin-bindende Rinder-Globulin kann aus Rinderseren in an sich bekannter Weise gewonnen werden. Bevorzugt enthält die erfindungsgemäß verwendete Standardlösung 5 bis 30 µg/ml Rinder-TBG. Besonders bevorzugt wird Rinder-TBG in physiologischer Menge eingesetzt, die im Bereich von 9 bis 20 µg/ml TBG liegt, wie es beispielsweise den Literaturstellen Lab. med. 6 (1982), 27-29, und J. Clin. Chem. Clin. Biochem. 23 (1985) 117-127, zu entnehmen ist.

Bei Verwendung zur Bestimmung von Thyroxin enthält die Standardlösung weiterhin Thyroxin. Das Thyroxin ist bevorzugt in einer Menge von 5 bis 500 ng/ml enthalten. Besonders bevorzugt wird eine physiologische Menge an Thyroxin der Standardlösung zugesetzt, d.h. eine Menge im Bereich von 5 bis 300 ng/ml.

Bei Verwendung zur Bestimmung von Trijodthyronin enthält die Standardlösung zusätzlich noch Trijodthyronin. Das Trijodthyronin wird bevorzugt in einer Menge von 0,5 bis 12 ng/ml eingesetzt. Besonders bevorzugt wird Trijodthyronin in physiologischer Menge, d.h. im Bereich von 0,5 bis 8 ng/ml zugegeben.

Die für das erfindungsgemäße Verfahren verwendete Standardlösung enthält außer Rinder-TBG und Thyroxin bzw. Trijodthyronin in Pufferlösung vorzugsweise noch Albumin, das die Stabilität der Lösung verbessert. Dabei ist es nicht erforderlich, der erfindungsgemäß bevorzugten Standardlösung Humanserumalbumin zuzusetzen, das natürlich geeignet ist. Ebenso geeignet sind auch die überall leicht erhältlichen und wesentlich günstigeren Albumine, beispielsweise aus Rinder- und Pferdeserum. Bevorzugt wird als Albumin Humanserum-, Rinderserum- oder Pferdeserumalbumin und insbesondere Rinderserumalbumin verwendet. Das Albumin wird bevorzugt in einer Menge von 40 bis 80 mg/ml Lösung verwendet. Besonders bevorzugt wird Albumin in physiologischer Menge, die im Bereich von 50 bis 70 mg/ml liegt, verwendet.

Zur Herstellung der Standardlösung wird Rinder-TBG und gegebenenfalls Albumin in einer Pufferlösung gelöst. Als Puffersystem sind alle Puffer geeignet, die einen pH-Bereich von 6,0 bis 8,0 haben, bevorzugt solche mit einem pH-Wert von 6,5 bis 7,5. Bevorzugt werden GOOD-Puffer eingesetzt, beispielsweise HEPES- oder TRIS-Puffer. Die Pufferkonzentration ist nicht kritisch, bevorzugt wird der Puffer in einer Konzentration von 30 bis 150 mmol/l verwendet. Besonders bevorzugt enthält die Standardlösung 50 bis 100 mmol/l Puffer.

Dieser Lösung wird sodann das Thyroxin oder Trijodthyronin in den geeigneten Konzentrationen zugesetzt. Dabei stellt sich dann ein Gleichgewicht zwischen proteingebundenem und freiem Thyreoidhormon ein analog dem in vivo im Blut herrschenden Gleichgewicht. Diese Lösung ist aufgrund ihrer Zusammensetzung auch bei längerer Lagerung stabil. Da sie aus standardisierten Einzelsubstanzen hergestellt wurde, weist sie eine stets gleichmäßige Zusammensetzung auf und liefert daher reproduzierbare Werte.

Zur Erstellung einer Eichkurve werden mehrere Standardlösungen - in der Regel vier bis sechs - verwendet, deren Thyreoidhormongehalte sich unterscheiden, um aus den erhaltenen Werten eine Kurve zeichnen zu können. In einer bevorzugten Ausführungsform des erfindungsgemäßen Verfahrens wird als erste Standardlösung eine Lösung verwendet, die nur Rinder-TBG und gegebenenfalls Albumin gelöst in einer Pufferlösung, aber kein Thyreoidhormon enthält. Zur weiteren Kalibrierung werden dann Lösungen mit definierten Hormongehalten eingesetzt.

Ein weiterer Gegenstand der Erfindung ist eine Standardlösung für die Bestimmung von Thyroxin oder Trijodthyronin, die Rinder-TBG und Thyroxin bzw. Trijodthyronin gelöst in einem Puffersystem enthält.

Bevorzugt enthält die Standardlösung 5 bis 30 µg/ml Rinder-TBG. Besonders bevorzugt sind in der Standardlösung physiologische Menge an Rinder-TBG, d.h. 9 bis 20 µg/ml vorhanden.

In einer bevorzugten Ausführungsform enthält die Standardlösung zur Stabilisierung weiterhin Albumin. Das Albumin ist dabei vorzugsweise in einer Menge von 40 bis 80 mg/ml enthalten. Besonders bevorzugt werden physiologische Mengen an Albumin, d.h. im Bereich von 50 bis 70 mg/ml Albumin, eingesetzt.

Die Standardlösung wird hergestellt, indem man Rinder-TBG und gegebenenfalls Albumin in einem Puffersystem löst und die jeweils gewünschte Menge an Thyroxin oder Trijodthyronin zugibt. Dabei liegt die Menge an Thyroxin bevorzugt im Bereich von 5 bis 500 ng/ml und besonders bevorzugt im Bereich der physiologischen Menge von 5 bis 300 ng/ml. Wird die Standardlösung zur Bestimmung von Trijodthyronin verwendet, so enthält sie Trijodthyronin bevorzugt in einer Menge von 0,5 bis 12 ng/ml und besonders bevorzugt in physiologischer Menge im Bereich von 0,5 bis 8 ng/ml.

Das Puffersystem weist geeigneterweise einen pH im Bereich von 6,0 bis 8,0 auf und ist bevorzugt ein GOOD-Puffer.

Erfindungsgemäß wird eine Standardlösung mit überlegener Stabilität zur Verfügung gestellt, die in einfacher Weise aus leicht erhältlichen Ausgangsmaterialien hergestellt werden kann.

### Beispiel 1

### Gewinnung von Rinder-TBG

Rinderplasma wird auf 2 mol/l Ammoniumsulfat gebracht, der Überstand abdekantiert auf 1 mol/l Ammoniumsulfat verdünnt und auf eine Chromatographiesäule (Sepharose, an die T₄ gebunden ist) aufgegeben. Es wird nacheinander mit:
1. 50 mmol/l Tris pH 7,5, 100 mmol/l Natriumchlorid
2. 50 mmol/l Tris pH 7,5, 750 mmol/l Natriumchlorid
3. 50 mmol/l Tris pH 7,5, 100 mmol/l Natriumchlorid
4. 50 mmol/l Tris pH 7,5, 100 mmol/l Natriumchlorid
   5 mmol/l 8-Anilino-Naphtalin-Sulfonsäure
gewaschen. Das Eluat wird in einer osmotischen Zelle konzentriert und 8-Aninilino-Naphtalin-Sulfonsäure durch Dialyse gegen 5 mmol/l Natriumphosphat pH 7,5, 10 mmol/l Natriumchlorid über zwei Tage entfernt.

### Beispiel 2

### Stabilität von T₃/T₄-Kalibratoren

Zusammensetzung der Kalibratoren:
6 % Rinderserumalbumin
50 mmol/l Hepespuffer, pH 7,4
15 µg/ml Rinder-TBG

FT₃-Gehalt 0, 2, 7, 15, 30 pg/ml
bzw. FT₄-Gehalt: 1, 10, 20, 40, 80 pg/ml

Die nachfolgende Tabelle 1 zeigt die Stabilität von T₃/T₄-Kalibratoren.

**Tabelle 1**

| Rezeptur Standard | Lagerungszeit bei 4 - 8°C (Monate) | Wiederfindung¹⁾ bei Analytgehalt 2 - 30 pg/ml | |
|---|---|---|---|
| | | T₃ | T₄ |
| ohne TBG | 3 | 118 | 110 |
| | 6 | 130 | 120 |
| | 12 | - | 130 |
| | | | |
| mit Human-TBG | 3 | 115 | 110 |
| | 6 | 130 | 120 |
| | 12 | 138 | 130 |
| | | | |
| mit Rinder-TBG | 3 | 100 | 100 |
| | 6 | 97 | 98 |
| | 12 | 99 | 97 |

| | | | |
|---|---|---|---|
| ¹⁾ Die Wiederfindung gibt den gefundenen Meßwert für T₃ bzw. T₄ in bezug auf den Meßwert für T₃ bzw. T₄ zu Beginn der Lagerung (100 %) an. | | | |

## Patentansprüche

1. Verfahren zur Bestimmung von Thyroxin (T₄) oder Trijodthyronin (T₃) im Serum, wobei man zur Eichung eine Standardlösung verwendet, die Thyroxin-bindendes Globulin (TBG) und Thyroxin bzw. Trijodthyronin gelöst in einer Pufferlösung enthält,
**dadurch gekennzeichnet,**
daß man Rinder-TBG verwendet.

2. Verfahren nach Anspruch 1,
**dadurch gekennzeichnet,**
daß als weitere Standardlösung eine Lösung verwendet wird, die Rinder-TBG gelöst in einer Pufferlösung, aber kein Thyroxin oder Trijodthyronin enthält.

3. Verfahren nach Anspruch 1 oder 2,
**dadurch gekennzeichnet,**
daß der Standardlösung zusätzlich Albumin zugesetzt wird.

4. Verfahren nach Anspruch 3,
**dadurch gekennzeichnet,**
daß als Albumin Rinderserumalbumin verwendet wird.

5. Verfahren nach Anspruch 3 oder 4,
**dadurch gekennzeichnet,**
daß man eine Standardlösung verwendet, die 40 bis 80 mg/ml und insbesondere 50 bis 70 mg/ml Albumin enthält.

6. Verfahren nach einem der vorhergehenden Ansprüche,
**dadurch gekennzeichnet,**
daß man eine Standardlösung verwendet, die 5 bis 30 µg/ml Rinder-TBG enthält.

7. Verfahren nach einem der vorhergehenden Ansprüche,
**dadurch gekennzeichnet,**
daß man eine Standardlösung verwendet, die 9 bis 20 µg/ml Rinder-TBG enthält.

8. Verfahren nach einem der Ansprüche 1 oder 3 bis 7,
**dadurch gekennzeichnet,**
daß man eine Standardlösung verwendet, die 5 bis 500 ng/ml und insbesondere 5 bis 300 ng/ml Thyroxin enthält.

9. Verfahren nach einem der Ansprüche 1 oder 3 bis 7,
**dadurch gekennzeichnet,**
daß man eine Standardlösung verwendet, die 0,5 bis 12 ng/ml und insbesondere 0,5 bis 8 ng/ml Trijodthyronin enthält.

10. Verfahren nach einem der vorhergehenden Ansprüche,
**dadurch gekennzeichnet,**
daß als Puffersystem ein GOOD-Puffer oder ein anderer Puffer mit einem pH-Bereich von 6 bis 8 verwendet wird.

11. Verfahren nach einem der vorhergehenden Ansprüche,
**dadurch gekennzeichnet,**
daß eine Pufferlösung verwendet wird, die 30 bis 150 mmol/l Puffer enthält.

12. Standardlösung für die Bestimmung von Thyroxin oder Trijodthyronin, enthaltend Rinder-TBG und Thyroxin oder Trijodthyronin gelöst in einer Pufferlösung.

13. Standardlösung nach Anspruch 12,
**dadurch gekennzeichnet,**
daß sie 5 bis 30 µg/ml Rinder-TBG und 30 bis 150 mmol/l Puffer enthält.

14. Standardlösung nach Anspruch 12 oder 13,
**dadurch gekennzeichnet,**
daß sie zusätzlich Albumin enthält.

15. Standardlösung nach Anspruch 14,
**dadurch gekennzeichnet,**
daß sie zusätzlich 40 bis 80 mg/ml Albumin enthält.

16. Standardlösung nach einem der Ansprüche 12 bis 15,
**dadurch gekennzeichnet,**
daß sie 5 bis 500 ng/ml Thyroxin und insbesondere 5 bis 300 ng/ml Thyroxin enthält.

17. Standardlösung nach einem der Ansprüche 13 bis 15,
**dadurch gekennzeichnet,**
daß sie 0,5 bis 12 ng/ml Trijodthyronin und insbesondere 0,5 bis 8 ng/ml Trijodthyronin enthält.

## Claims

1. Method for the determination of thyroxine (T₄) or triiodothyronine (T₃) in serum whereby a standard solution is used for the calibration which contains thyroxine-binding globulin (TBG) and thyroxine or triiodothyronine dissolved in a buffer solution,
**wherein**
bovine TBG is used.

2. Method as claimed in claim 1,
**wherein**
a solution is used as a further standard solution which contains bovine TBG dissolved in a buffer solution but contains no thyroxine or triiodothyronine.

3. Method as claimed in claim 1 or 2,
**wherein**
albumin is in addition added to the standard solution.

4. Method as claimed in claim 3,
**wherein**
bovine serum albumin is used as albumin.

5. Method as claimed in claim 3 or 4,
**wherein**
a standard solution is used which contains 40 to 80 mg/ml and in particular 50 to 70 mg/ml albumin.

6. Method as claimed in one of the previous claims,
**wherein**
a standard solution is used which contains 5 to 30 µg/ml bovine TBG.

7. Method as claimed in one of the previous claims,
**wherein**
a standard solution is used which contains 9 to 20 µg/ml bovine TBG.

8. Method as claimed in one of the claims 1 or 3 to 7,
**wherein**
a standard solution is used which contains 5 to 500 ng/ml and in particular 5 to 300 ng/ml thyroxine.

9. Method as claimed in one of the claims 1 or 3 to 7,
**wherein**
a standard solution is used which contains 0.5 to 12 ng/ml and in particular 0.5 to 8 ng/ml triiodothyronine.

10. Method as claimed in one of the previous claims,
**wherein**
a GOOD buffer or another buffer with a pH range from 6 to 8 is used as the buffer system.

11. Method as claimed in one of the previous claims,
**wherein**
a buffer solution is used which contains 30 to 150 mmol/l buffer.

12. Standard solution for the determination of thyroxine or triiodothyronine containing bovine TBG and thyroxine or triiodothyronine dissolved in a buffer solution.

13. Standard solution as claimed in claim 12,
**wherein**
it contains 5 to 30 µg/ml bovine TBG and 30 to 150 mmol/l buffer.

14. Standard solution as claimed in claim 12 or 13,
**wherein**
it in addition contains albumin.

15. Standard solution as claimed in claim 14,
**wherein**
it in addition contains 40 to 80 mg/ml albumin.

16. Standard solution as claimed in one of the claims 12 to 15,
**wherein**
it contains 5 to 500 ng/ml thyroxine and in particular 5 to 300 ng/ml thyroxine.

17. Standard solution as claimed in one of the claims 13 to 15,
**wherein**
it contains 0.5 to 12 ng/ml triiodothyronine and in particular 0.5 to 8 ng/ml triiodothyronine.

## Revendications

1. Procédé de détermination de la thyroxine (T₄) ou de la tri-iodothyronine (T₃) dans du sérum, dans lequel on utilise pour l'étalonnage une solution étalon qui contient de la globuline fixant la thyroxine (TBG) et de la thyroxine ou de la tri-iodothyronine dissoutes dans une solution tampon, caractérisé par le fait que l'on utilise de la TBG bovine.

2. Procédé selon la revendication 1, caractérisé par le fait qu'en tant qu'autre solution étalon, on utilise une solution qui contient de la TBG bovine dissoute dans une solution tampon. mais pas de thyroxine ou de tri-iodothyronine.

3. Procédé selon la revendication 1 ou 2, caractérisé par le fait qu'à la solution étalon, on ajoute en outre de l'albumine.

4. Procédé selon la revendication 3, caractérisé par le fait que l'albumine utilisée est l'albumine de sérum bovin.

5. Procédé selon la revendication 3 ou 4, caractérisé par le fait que l'on utilise une solution étalon contenant de 40 à 80 mg/ml et en particulier. de 50 à 70 mg/ml d'albumine.

6. Procédé selon l'une quelconque des revendications précédentes, caractérisé par le fait que l'on utilise une solution étalon contenant de 5 à 30 µg/ml de TBG bovine.

7. Procédé selon l'une quelconque des revendications précédentes, caractérisé par le fait que l'on utilise une solution étalon contenant de 9 à 20 µg/ml de TBG bovine.

8. Procédé selon l'une quelconque des revendications 1 ou 3 à 7, caractérisé par le fait que l'on utilise une solution étalon contenant de 5 à 500 ng/ml et en particulier, de 5 à 300 ng/ml de thyroxine.

9. Procédé selon l'une quelconque des revendications 1 ou 3 à 7, caractérisé par le fait que l'on utilise une solution étalon contenant de 0,5 à 12 ng/ml et en particulier, de 0,5 à 8 ng/ml de tri-iodothyronine.

10. Procédé selon l'une quelconque des revendications précédentes. caractérisé par le fait qu'en tant que système tampon, on utilise un tampon GOOD ou un autre tampon ayant un pH dans la gamme de 6 à 8.

11. Procédé selon l'une quelconque des revendications précédentes. caractérisé par le fait que l'on utilise une solution tampon contenant de 30 à 150 mmoles/l de tampon.

12. Solution étalon destinée à la détermination de thyroxine ou de tri-iodothyronine, contenant de la TBG bovine et de la thyroxine ou de la tri-iodothyronine dissoutes dans une solution tampon.

13. Solution étalon selon la revendication 12, caractérisée par le fait qu'elle contient de 5 à 30 µg/ml de TBG bovine et de 30 à 150 mmoles/l de tampon.

14. Solution étalon selon la revendication 12 ou 13, caractérisée par le fait qu'elle contient en outre de l'albumine.

15. Solution étalon selon la revendication 14, caractérisée par le fait qu'elle contient en outre de 40 à 80 mg/ml d'albumine.

16. Solution étalon selon l'une quelconque des revendications 12 à 15, caractérisée par le fait qu'elle contient de 5 à 500 ng/ml de thyroxine et en particulier, de 5 à 300 ng/ml de thyroxine.

17. Solution étalon selon l'une quelconque des revendications 13 à 15, caractérisée par le fait qu'elle contient de 0,5 à 12 ng/ml de tri-iodothyronine et en particulier, de 0,5 à 8 ng/ml de tri-iodothyronine.
